# EUROPÄISCHE PATENTANMELDUNG

(11) **EP 2 548 812 A1**
(43) Veröffentlichungstag der Anmeldung: **23.01.2013**
(21) Anmeldenummer: 12005114.9
(22) Anmeldetag: 11.07.2012
(51) Int. Cl.: B65D 75/24, B65D 81/32, B65D 75/32, B65B 9/04, A61F 13/551

(54) **Mehrfachfaltpackung und Verpackungsmaschine zu deren Herstellung**

(30) Priorität: 18.07.2011 DE 202011103391 U
(71) Anmelder: Multivac Sepp Haggenmüller GmbH & Co. KG, 87787 Wolfertschwenden (DE)
(72) Erfinder: Mondry, Hans, 87439 Kempten (DE)
(74) Vertreter: Grünecker, Kinkeldey, Stockmair & Schwanhäusser

(57) **Zusammenfassung**

Die vorliegende Erfindung bezieht sich auf eine Mehrfachfaltpackung (1), die zwei Faltlinien (9, 10) aufweist, so dass die Mehrfachfaltpackung (1) einen zwischen den beiden Faltlinien (9, 10) befindlichen Standbereich (8) sowie einen ersten (6) und einen zweiten Teilbereich (7) umfasst, wobei jeder Teilbereich (6, 7) ein oder mehrere Fächer (2, 2a) aufweist, sowie auf eine Verpackungsmaschine zum Herstellen einer solchen Verpackung.

## Beschreibung

Die vorliegende Erfindung bezieht sich auf eine Mehrfachfaltpackung gemäß dem Oberbegriff des Anspruchs 1, sowie auf eine Verpackungsmaschine zum Herstellen einer solchen Verpackung.

Mehrfachfaltpackungen sind bekannt. Diese Verpackungen verfügen über mehrere Fächer ― daher der Name Mehrfachpackung. In jedem Fach der Mehrfachpackung kann ein einzelnes Produkt verpackt sein, beispielsweise eine Portion eines Lebensmittels. Bekannt ist es aus der Praxis auch, solche Mehrfachpackungen zu falten und dadurch eine Mehrfachfaltpackung zu bilden. Durch das Falten können die äußeren Abmessungen der Verpackung reduziert werden, beispielsweise um die Verpackung besser in einer Umverpackung wie einem Karton unterbringen zu können.

Als nachteilig hat sich unter gewissen Umständen die Handhabung solcher Mehrfachfaltpackungen erwiesen. Die Aufgabe der vorliegenden Erfindung ist es, diese Handhabung mit konstruktiv möglichst einfachen Mitteln zu verbessern.

Diese Aufgabe wird gelöst durch eine Mehrfachfaltpackungen mit den Merkmalen des Anspruchs 1 beziehungsweise durch eine Verpackungsmaschine zum Herstellen einer solchen Mehrfachfaltpackung.

Die erfindungsgemäße Mehrfachfaltpackung umfasst einen ersten und einen zweiten Teilbereich, die jeweils ein oder mehrere Fächer aufweisen. Zwischen diesen beiden Teilbereichen befindet sich ein Standbereich. Zwischen dem Standbereich und jedem der beiden seitlichen Teilbereiche befindet sich jeweils eine Faltlinie, um die die Mehrfachfaltpackung gefaltet ist. Der Vorteil der erfindungsgemäßen Mehrfachfaltpackung besteht darin, dass der zwischen den beiden Faltlinien gebildete Standbereich eine zweidimensionale Standfläche für die Mehrfachfaltpackung definiert, so dass die Mehrfachfaltpackung stabil aufgestellt und in diesem aufgestellten Zustand einem Kunden präsentiert werden kann, ohne dass die Gefahr besteht, dass die Mehrfachfaltpackung umkippt oder an einer horizontalen Perforationslinie umknickt.

Die Stabilität der Mehrfachfaltpackung wird besonders groß und ihre Herstellung wird besonders einfach, wenn die Faltlinien parallel zueinander verlaufen. Denkbar wäre es jedoch auch, dass die beiden Faltlinien unter einem Winkel zueinander verlaufen, um geometrisch ungewöhnliche und daher beim Kunden noch mehr Aufmerksamkeit erregende Verpackungsformen zu erzielen. Möglich wäre es darüber hinaus auch, mehr als lediglich zwei Faltlinien vorzusehen.

Die Stabilität der Mehrfachfaltpackung kann weiter dadurch verbessert werden, wenn jeder Teilbereich gleich viele Fächer aufweist. Diese Fächer können sich einreihig oder mehrreihig auf beiden Seiten des Standbereichs erstrecken.

Vorzugsweise ist jeder Teilbereich der Mehrfachfaltpackung gegenüber dem Standbereich jeweils um etwa 90° schwenkbar. Dies bewirkt, dass die beiden Teilbereiche zumindest annähernd vertikal ausgerichtet sind, wenn sich der flächige Standbereich auf einem ebenen, horizontalen Untergrund befindet.

Um die Stabilität der Mehrfachfaltpackung beim Aufstellen weiter zu erhöhen, ist es günstig, wenn sich der Standbereich mittig auf der Mehrfachfaltpackung befindet.

In einem Aufstellzustand der Mehrfachfaltpackung, in dem die Faltpackung beispielsweise mit ihrem Standbereich auf einem ebenen Untergrund aufgestellt ist, sollte sich der erste Teilbereich zweckmäßig parallel zum zweiten Teilbereich befinden. Dies verhindert ein Moment, welches die Faltpackung zum Kippen bringen könnte.

Besonders stabil wird die erfindungsgemäße Mehrfachfaltpackung jedoch, wenn sich in einem Aufstellzustand ein Schwerpunkt der Faltpackung vertikal oberhalb des Standbereichs befindet, sobald dieser Standbereich auf einem horizontalen Untergrund angeordnet ist. Insbesondere könnte sich sogar ein Schwerpunkt der mit einem Produkt befüllten Mehrfachfaltpackung vertikal oberhalb dieses Standbereichs befinden. Sofern keine weiteren äußeren Einflüsse herrschen, ist damit ein Umkippen der Mehrfachfaltpackung ausgeschlossen.

Bevorzugt ist entweder im Standbereich der Faltpackung gar keine Oberfolie vorhanden, oder im Standbereich liegt die Oberfolie flächig auf der Unterfolie auf. Beides erleichtert die Ausbildung von Falt- oder Knicklinien, da die Mehrfachfaltpackung im Standbereich beziehungsweise an den beiden äußeren Kanten des Standbereichs besonders flach wird.

Die Handhabung der Mehrfachfaltpackung kann weiter verbessert werden, wenn mindestens eine der beiden Faltlinien eine Perforation aufweist. Diese Perforation ermöglicht es, die beiden Teilbereiche der Mehrfachfaltpackung voneinander zu trennen, um die darin aufgenommenen Produkte getrennt voneinander entnehmen oder lagern zu können.

In einer weiteren Variante wäre es sogar denkbar, dass zwischen allen benachbarten Fächern der Mehrfachfaltpackung jeweils eine Perforation vorgesehen ist. Auf diese Weise könnten sämtliche Fächer voneinander vereinzelt werden.

Je nach Art der verwendeten Verpackungsfolien hat es sich als günstig herausgestellt, wenn zwischen zwei benachbarten Löchern der Perforation Stege mit einer Breite von 0,5 bis 1,0 mm vorgesehen sind, insbesondere mit einer Breite von etwa 0,7 mm. Stege mit solchen Abmessungen verhindern ein ungewolltes Trennen benachbarter Fächer, ermöglichen aber dennoch ein gezieltes Trennen benachbarter Fächer ohne allzu großen Aufwand.

Die Funktionalität der erfindungsgemäßen Mehrfachfaltpackung kann weiter dadurch verbessert werden, dass ein Verschluss zum lösbaren Halten der Mehrfachfaltpackung in einem geschlossenen Zustand beziehungsweise Aufstellzustand vorgesehen ist. Dieser Verschluss verhindert, dass sich die Mehrfachfaltpackung ungewollt vom Aufstellzustand in einen geöffneten Zustand begibt. Besonders vorteilhaft ist solch ein Verschluss, wenn er wiederverschließbar ist.

Der Verschluss könnte insbesondere mindestens ein mechanisches Verschlusselement umfassen. Denkbar wäre beispielsweise ein Rast- oder Klettverschluss. Günstiger für die Herstellung ist es jedoch, wenn das Verschlusselement einen Noppen auf einem der beiden Teilbereiche sowie eine dem Noppen zugeordnete Aussparung auf dem anderen der beiden Teilbereiche aufweist. Auf diese Weise bildet sich ein Druckknopfverschluss, der beim Tiefziehen der Verpackungsfolien leicht hergestellt werden kann. Selbstverständlich könnten auch mehrere Noppen und eine passende Anzahl von Aussparungen vorgesehen werden.

In einer bevorzugten Variante kann als Verschlusselement der Mehrfachfaltpackung mindestens ein Etikett vorgesehen sein, das beim Öffnen entweder eingerissen oder abgezogen werden kann.

Die Erfindung bezieht sich auch auf eine Verpackungsmaschine zum Herstellen einer solchen Mehrfachfaltpackung. Diese Verpackungsmaschine umfasst eine Einrichtung zum Erzeugen zweier Faltlinien in der Mehrfachfaltpackung, insbesondere zweier paralleler Faltlinien, so dass zwischen diesen Faltlinien der Standbereich gebildet werden kann.

Wenn in den Faltlinien eine Perforation vorgesehen werden soll, sind zum Einbringen dieser Perforation vorzugsweise zwei parallel angeordnete Stanzmesser oder Rotationsmesser vorhanden.

Die Herstellung und Handhabung der Mehrfachfaltpackung kann noch weiter dadurch vereinfacht werden, wenn die Verpackungsmaschine ein Umfaltwerkzeug zum automatischen Umfalten der ersten und zweiten Teilbereiche der Mehrfachfaltpackung um die Faltlinien aufweist.

Im Folgenden wird ein vorteilhaftes Ausführungsbeispiel der Erfindung anhand einer Zeichnung näher dargestellt. Im Einzelnen zeigen:
- Figur 1: eine perspektivische Ansicht einer Mehrfachfaltpackung in einer geöffneten Stellung,
- Figur 2: eine Seitenansicht der Mehrfachfaltpackung im geöffneten Zustand,
- Figur 3: eine Seitenansicht der Mehrfachfaltpackung im geschlossen oder Aufstellzustand mit einer vergrößerten Darstellung des Verschlussbereichs,
- Figur 4: eine Seitenansicht der Mehrfachfaltpackung in einer auf einem Untergrund aufgestellten Situation,
- Figur 5: eine perspektivische Ansicht der Mehrfachfaltpackung im geschlossenen Zustand,
- Figur 6: eine perspektivische Ansicht einer alternativen Mehrfachfaltpackung in einer geöffneten Stellung,
- Figur 7: die alternative Mehrfachfaltpackung in einer Seitenansicht im geöffneten Zustand,
- Figur 8: eine Seitenansicht der alternativen Mehrfachfaltpackung im geschlossen Zustand, und
- Figur 9: eine Seitenansicht der alternativen Mehrfachfaltpackung in einer auf einem Untergrund aufgestellten Situation im geschlossen Zustand.

Gleiche Komponenten sind in den Figuren durchgängig mit gleichen Bezugszeichen versehen.

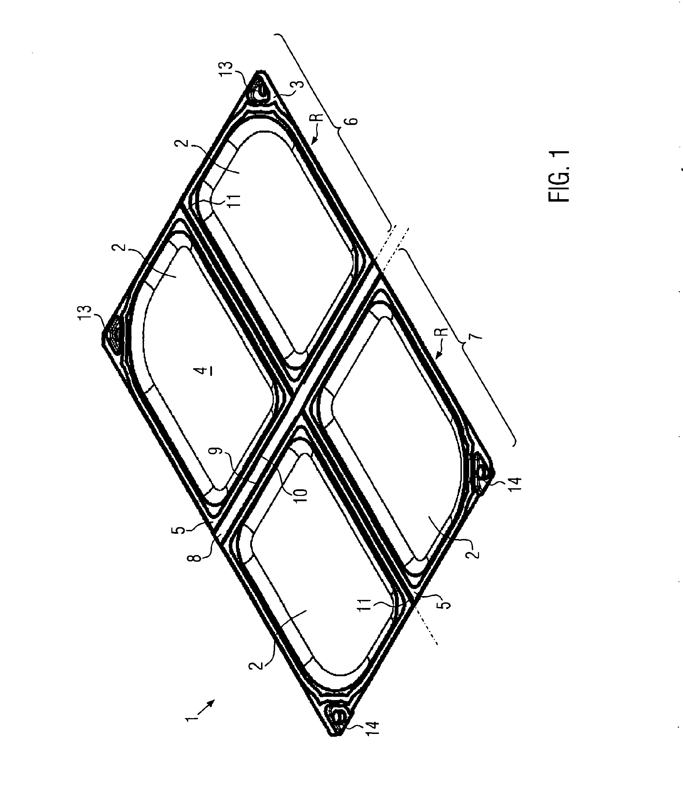

Figur 1 zeigt ein Ausführungsbeispiel einer erfindungsgemäßen Mehrfachfaltpackung 1. Die Mehrfachfaltpackung oder im Folgenden kurz Verpackung 1 verfügt über eine Gruppe von vier Fächern 2, die jeweils eine Portion eines Produkts, beispielsweise eines Lebensmittels, aufnehmen können. Die Fächer 2 sind im dargestellten Ausführungsbeispiel in zwei Reihen R mit jeweils zwei hintereinander liegenden Fächern 2 ausgebildet. Die Fächer selbst sind durch Tiefziehen in einer vergleichsweise stabilen Unterfolie 3 ausgebildet. Eine transparente und daher in Figur 1 nicht erkennbare Oberfolie 4 ist so auf die Unterfolie 3 aufgesiegelt, dass sie jedes Fach 2 in der gemeinsamen Ebene der Ränder 5 aller Fächer 2 hermetisch verschließt. Sowohl die Unterfolie 3, als auch die Oberfolie 4 erstrecken sich also durchgängig beziehungsweise einstückig über die gesamte, in Figur 1 gezeigte Mehrfachfaltpackung 1.

Die eine Reihe R von Fächern 2 bildet einen ersten Teilbereich 6 der Mehrfachfaltpackung, während die zweite Reihe R von Fächern 2 einen zweiten Teilbereich 7 der Mehrfachfaltpackung 1 bildet. Jeder der beiden Teilbereiche 6, 7 umfasst also im dargestellten Ausführungsbeispiel zwei Fächer 2. Zwischen den beiden Teilbereichen 6, 7 befindet sich mittig auf der Mehrfachfaltpackung 1 ein Standbereich 8. Er ist im dargestellten Ausführungsbeispiel rechteckförmig und befindet sich zwischen zwei parallelen Faltlinien 9, 10, die zwischen dem Standbereich 8 und dem jeweiligen ersten oder zweiten Teilbereich 6, 7 liegen. Entlang der Faltlinie 9, 10 kann die Mehrfachfaltpackung 1 geknickt werden. Dies kann erleichtert werden, indem die Faltlinien 9, 10 eine Materialschwächung und/oder eine Perforation aufweisen.

Eine solche Perforation, die beispielsweise durch Stege beabstandete Löcher aufweisen kann, kann dazu verwendet werden, die Fächer 2 des ersten Teilbereichs 6 leichter von denen des zweiten Teilbereichs 7 zu trennen. Darüber hinaus kann eine Querperforation 11 auch zwischen benachbarten Fächern 2 eines einzelnen Teilbereichs 6 oder 7 vorgesehen sein, um die Fächer 2 des Teilbereichs 6, 7 voneinander vereinzeln zu können.

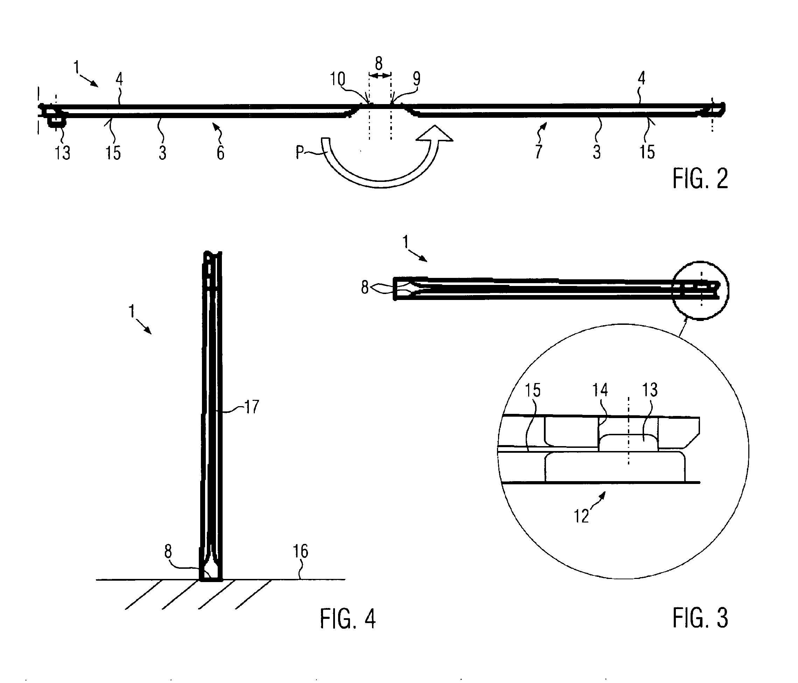

Jeweils in den vier äußeren Ecken der Mehrfachfaltpackung 1 befinden sich Verschlusselemente, um einen lösbaren und wiederverschließbaren Verschluss 12 (siehe Figur 3) für die Verpackung 1 zu bilden. Bei zweien dieser Verschlusselemente handelt es sich um Verschlussnoppen 13, die nach unten vom Boden der Mehrfachfaltpackung 1 abstehen. Bei den beiden anderen Verschlusselementen handelt es sich um Aussparungen 14, die jeweils zur engen Aufnahme eines Verschlussnoppens 13 ausgebildet sind. Während sich die beiden Verschlussnoppen 13 an den äußeren Ecken des einen Teilbereichs 6 befinden, befinden sich die Aussparungen 14 an den äußeren Ecken des anderen Teilbereichs 7 der Verpackung 1.

Figur 2 zeigt eine Seitenansicht der Mehrfachfaltpackung 1. Wie in Figur 1 befindet sich die Verpackung 1 dabei in einer geöffneten Stellung, in der die Fächer 2 der beiden Teilbereiche 6, 7 in einer gemeinsamen Ebene liegen. Zu erkennen ist hier nun insbesondere die in einer durchgehenden Ebene aufgespannte Oberfolie 4. Zu erkennen ist auch, dass die Unterfolie 3 im Standbereich 8, d.h. zwischen den beiden Faltlinien 9, 10, nicht tiefgezogen ist. In diesem Standbereich 8 liegt die Oberfolie 4 daher flächig auf der Unterfolie 3 auf, so dass die Verpackung 1 in diesem Bereich vergleichsweise flach ist. Dies ermöglicht es ― zusammen mit der Materialschwächung oder der Perforation der Verpackung 1 im Bereich der Faltlinien 9, 10 ―, die Verpackung 1 in der durch den Pfeil P angegeben Richtung zu falten. Dabei wird der erste, in Figur 2 links dargestellte Teilbereich 6 entgegen dem Uhrzeigersinn um 90° um die Faltlinie 10 heruntergeschwenkt. Der zweite, rechts dargestellte Teilbereich 7 hingegen wird im Uhrzeigersinn um die Faltlinie 9 heruntergeschwenkt.

Das Ergebnis dieses Vorgangs ist in Figur 3 zu sehen, in dem sich die Mehrfachfaltpackung 1 in einem geschlossenen beziehungsweise einem Aufstellzustand befindet. In diesem Zustand liegen die Fächer 2 der ersten und zweiten Teilbereiche 6, 7 mit ihren Böden 15 aneinander an. Die Breite des Standbereichs 8 entspricht etwa der doppelten Tiefe eines einzelnen Fachs 2, kann jedoch auch größer sein. In der Vergrößerung ist dargestellt, wie auf der dem Standbereich 8 gegenüberliegenden Seite der Mehrfachfaltpackung 1 ein Verschluss 12 gebildet wird, um die Verpackung 1 lösbar in der geschlossenen Stellung zu halten. Dieser Verschluss 12 wird dadurch erzielt, dass der über den Boden 15 hinaus nach unten vorstehende Vorsprung 13 (siehe Figur 2) durch das Falten der Verpackung 1 in Eingriff gelangt mit einer Aussparung 14. Diese Aussparung 14 ist so ausgebildet, dass der Vorsprung oder Verschlussnoppen 13 formschlüssig durch Reibung in der Aussparung 14 gehalten wird.

Figur 4 zeigt die Mehrfachfaltpackung 1 in einem Aufstellzustand. Dabei befindet sie sich im geschlossenen Zustand so auf einem ebenen, horizontalen Untergrund 16, dass der Standbereich 8 der Verpackung 1 flächig auf dem Untergrund 16 aufliegt. Die Mehrfachfaltpackung 1 ist dabei so ausgebildet, dass sich ihr Schwerpunkt 17 vertikal oberhalb des Standbereichs 8 befindet, vorzugsweise etwa mittig über dem Standbereich 8. Dabei wird eine besonders stabile Position der Mehrfachfaltpackung 1 erreicht, die sie gegen ein Verkippen schützt. In dieser aufgestellten Position kann die Mehrfachfaltpackung 1 demnach stabil gelagert und beispielsweise Verbrauchern präsentiert werden.

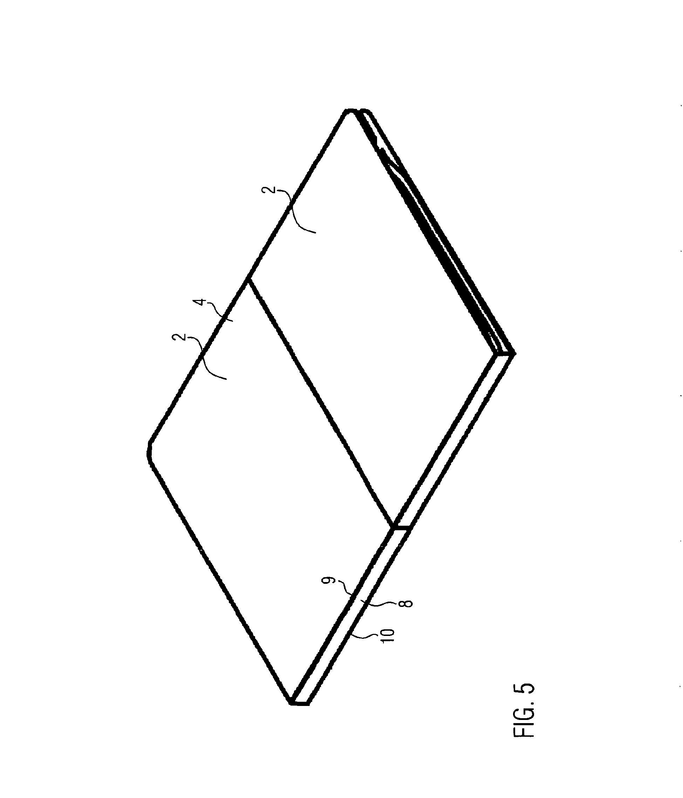

Figur 5 zeigt die Mehrfachfaltpackung 1 schließlich in einer perspektivischen Ansicht in ihrem geschlossen Zustand. Von oben sind die beiden Fächer 2 eines der beiden Teilbereiche 6, 7 zu sehen. Die Fächer der beiden Teilbereiche liegen flächig aneinander an, insbesondere an den Böden 15 der Fächer 2. Der Standbereich 8, der durch die beiden im 90°-Winkel abgewinkelten Faltlinien 9, 10 begrenzt wird, steht etwa senkrecht zu der durch die Oberfolie 4 gebildeten Ebene.

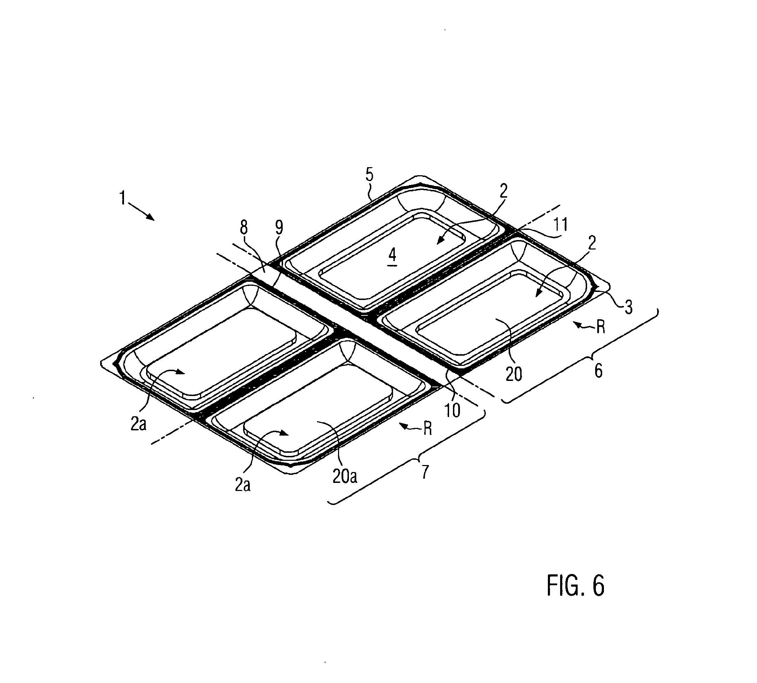

Figur 6 zeigt ein alternatives Ausführungsbeispiel einer erfindungsgemäßen Mehrfachfaltpackung 1. Die Mehrfachfaltpackung oder im Folgenden kurz Verpackung 1 verfügt über eine Gruppe von vier Fächern 2, 2a, die jeweils eine Portion eines Produkts, beispielsweise eines Lebensmittels, aufnehmen können. Die Fächer 2, 2a sind im dargestellten Ausführungsbeispiel in zwei Reihen R mit jeweils zwei hintereinander liegenden Fächern 2, 2a ausgebildet. Die Fächer selbst sind durch Tiefziehen in einer vergleichsweise stabilen Unterfolie 3 ausgebildet. Eine transparente und daher in Figur 6 nicht erkennbare Oberfolie 4 ist so auf die Unterfolie 3 aufgesiegelt, dass sie jedes Fach 2, 2a in der gemeinsamen Ebene der Ränder 5 aller Fächer 2, 2a hermetisch verschließt. Sowohl die Unterfolie 3, als auch die Oberfolie 4 erstrecken sich also durchgängig beziehungsweise einstückig über die gesamte, in Figur 6 gezeigte Mehrfachfaltpackung 1.

Die eine Reihe R von Fächern 2 bildet einen ersten Teilbereich 6 der Mehrfachfaltpackung, während die zweite Reihe R von Fächern 2a einen zweiten Teilbereich 7 der Mehrfachfaltpackung 1 bildet. Jeder der beiden Teilbereiche 6, 7 umfasst also im dargestellten Ausführungsbeispiel zwei Fächer 2, 2a. Zwischen den beiden Teilbereichen 6, 7 befindet sich mittig auf der Mehrfachfaltpackung 1 ein Standbereich 8. Er ist im dargestellten Ausführungsbeispiel rechteckförmig und befindet sich zwischen zwei parallelen Faltlinien 9, 10, die zwischen dem Standbereich 8 und dem jeweiligen ersten oder zweiten Teilbereich 6, 7 liegen. Entlang der Faltlinie 9, 10 kann die Mehrfachfaltpackung 1 geknickt werden. Dies kann erleichtert werden, indem die Faltlinien 9, 10 eine Materialschwächung und/oder eine Perforation aufweisen.

Eine solche Perforation, die beispielsweise durch Stege beabstandete Löcher aufweisen kann, kann dazu verwendet werden, die Fächer 2 des ersten Teilbereichs 6 leichter von denen des zweiten Teilbereichs 7 zu trennen. Darüber hinaus kann eine Querperforation 11 auch zwischen benachbarten Fächern 2 bzw. 2a eines einzelnen Teilbereichs 6 bzw. 7 vorgesehen sein, um die Fächer 2 bzw. 2a des Teilbereichs 6 bzw. 7 voneinander vereinzeln zu können.

Die Fächer 2 weisen einen nach unten geformten Packungsboden 20 auf und die Fächer 2a weisen einen dazu komplementären und nach oben, also in Richtung der Oberfolie 4, geformten Packungsboden 20a auf.

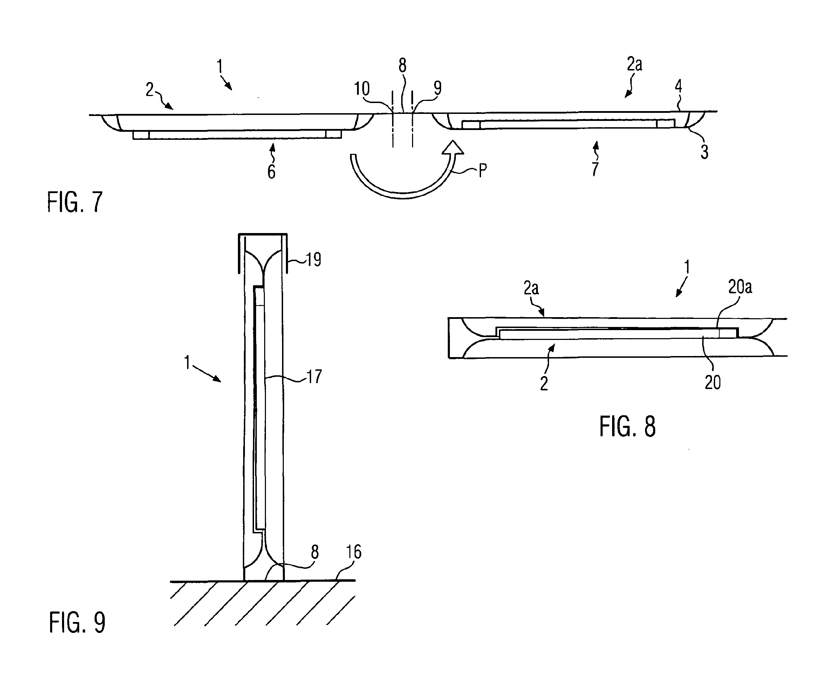

Figur 7 zeigt eine Seitenansicht der Mehrfachfaltpackung 1. Wie in Figur 6 befindet sich die Verpackung 1 dabei in einer geöffneten Stellung, in der die Fächer 2, 2a der beiden Teilbereiche 6, 7 in einer gemeinsamen Ebene liegen. Zu erkennen ist hier nun insbesondere die in einer durchgehenden Ebene aufgespannte Oberfolie 4. Zu erkennen ist auch, dass die Unterfolie 3 im Standbereich 8, d.h. zwischen den beiden Faltlinien 9, 10, nicht tiefgezogen ist. In diesem Standbereich 8 liegt die Oberfolie 4 daher flächig auf der Unterfolie 3 auf, so dass die Verpackung 1 in diesem Bereich vergleichsweise flach ist. Dies ermöglicht es ― zusammen mit der Materialschwächung oder der Perforation der Verpackung 1 im Bereich der Faltlinien 9, 10 ―, die Verpackung 1 in der durch den Pfeil P angegeben Richtung zu falten. Dabei wird der erste, in Figur 7 links dargestellte Teilbereich 6 entgegen dem Uhrzeigersinn um 90° um die Faltlinie 10 heruntergeschwenkt. Der zweite, rechts dargestellte Teilbereich 7 hingegen wird im Uhrzeigersinn um die Faltlinie 9 heruntergeschwenkt.

Das Ergebnis dieses Vorgangs ist in Figur 8 zu sehen, in dem sich die Mehrfachfaltpackung 1 in einem geschlossenen beziehungsweise einem Aufstellzustand befindet. In diesem Zustand liegen die Fächer 2 des ersten Teilbereichs 6 und die Fächer 2a des zweiten Teilbereichs 7 mit ihren Böden 20 und 20a aneinander an, d.h. die beiden Teilbereiche 6 und 7 greifen ineinander. Die Breite des Standbereichs 8 entspricht etwa der doppelten Tiefe eines einzelnen Fachs 2, 2a, kann jedoch auch größer sein.

Figur 9 zeigt die Mehrfachfaltpackung 1 in einem Aufstellzustand. Sie wird durch ein Etikett 19 an der dem Standbereich 8 gegenüberliegenden Seite zusammengehalten. Das Etikett 19 kann die Eigenschaft aufweisen, auch nach einem Abziehen durch eine entsprechende Klebeeigenschaft wiederverschließbar zu sein. Dabei befindet die Verpackung 1 sich im geschlossenen Zustand so auf einem ebenen, horizontalen Untergrund 16, dass der Standbereich 8 der Verpackung 1 flächig auf dem Untergrund 16 aufliegt. Die Mehrfachfaltpackung 1 ist dabei so ausgebildet, dass sich ihr Schwerpunkt 17 vertikal oberhalb des Standbereichs 8 befindet, vorzugsweise etwa mittig über dem Standbereich 8. Dabei wird eine besonders stabile Position der Mehrfachfaltpackung 1 erreicht, die sie gegen ein Verkippen schützt. In dieser aufgestellten Position kann die Mehrfachfaltpackung 1 demnach stabil gelagert und beispielsweise Verbrauchern präsentiert werden. Die ineinander greifenden Packungsböden 20 und 20a sind zueinander derart kongruent, dass sie im zusammengefügten Zustand der Verpackung 1 eine hohe Stabilität geben.

Ausgehend von dem dargestellten Ausführungsbeispiel kann die erfindungsgemäße Verpackung 1 in vielfacher Hinsicht verändert werden. Beispielsweise wäre es selbstverständlich denkbar, in jedem der beiden Teilbereich 6, 7 mehrere nebeneinander liegende Reihen R von Fächern 2 sowie lediglich ein Fach oder auch mehr Fächer 2 hintereinander vorzusehen. Auch die Zahl und Anordnung der Fächer 2 in den beiden Teilbereichen 6, 7 muss nicht unbedingt symmetrisch sein.

Denkbar wäre auch eine Variante, bei der die beiden Teilbereiche nicht mit den Böden 15 der Fächer 2, sondern mit den Oberfolien 4 aneinander gefaltet werden. Dies wäre der Fall, wenn in Figur 2 der rechte Teilbereich 7 gegen den Uhrzeigersinn nach oben und der linke Teilbereich 6 im Uhrzeigersinn ebenfalls nach oben gefaltet würden. Statt eines Verschlusses 12 mit mechanischen Verschlusselementen könnte dann beispielsweise ein Etikett die beiden Teilbereiche 6, 7 an dem freien, dem Standbereich 8 gegenüberliegenden Ende verbinden, um die Verpackung 1 in ihrem geschlossenen Zustand zu halten. Ebenso ist eine Verpackung denkbar, die anstatt einer ebenen Oberfolie 4 eine ähnlich oder gleich wie die Fächer 2, 2a geformte Oberfolie aufweist. Entsprechend kann auch die Verformung der Packungsböden 20, 20a in der geformten Oberfolie eingebracht sein, um beispielsweise zwei benachbarte Packungen 1 aneinander anliegen zu lassen, indem die Packungsböden der einen Verpackung mit der geformten Oberfolie der anderen Packung zusammenwirken.

Die Erfindung bezieht sich auch auf eine Verpackungsmaschine zum Herstellen der dargestellten Mehrfachfaltpackung. Diese Verpackungsmaschine kann beispielsweise in Form einer Tiefziehverpackungsmaschine ausgebildet sein, die die Mehrfachfaltpackung 1 jeweils in einem Hauptarbeitstakt produziert. Rotationsmesser können zum Erzeugen von Perforationen in Längsrichtung der tiefgezogenen Verpackungsfolie dienen, um die Faltlinien 9, 10 zu erzeugen. Ein quer zur Produktionsrichtung angeordnetes Stanzmesser kann dazu dienen, die Querperforationen 11 zu erzeugen. Nach dem Erzeugen der Perforationen wird die Mehrfachfaltpackung 1 vereinzelt. Ein nachfolgendes Umfaltwerkzeug kann dazu dienen, die beiden Teilbereiche 6, 7 der Verpackung 1 um die Faltlinien 9, 10 zu falten und den Verschluss 12 zu schließen und/oder das Etikette 19 anzubringen.

## Patentansprüche

1. Mehrfachfaltpackung (1) mit mehreren Fächern (2, 2a), wobei die Mehrfachfaltpackung (1) eine durchgehende, in den einzelnen Fächern (2, 2a) Verpackungsmulden ausbildende Unterfolie (3) und eine die Verpackungsmulden verschließende Oberfolie (4) aufweist,
**dadurch gekennzeichnet, dass** die Mehrfachfaltpackung (1) zwei Faltlinien (9, 10) aufweist, so dass die Mehrfachfaltpackung (1) einen zwischen den beiden Faltlinien (9, 10) befindlichen Standbereich (8) sowie einen ersten (6) und einen zweiten Teilbereich (7) umfasst, wobei jeder Teilbereich (6, 7) ein oder mehrere Fächer (2, 2a) aufweist.

2. Mehrfachfaltpackung nach Anspruch 1, **dadurch gekennzeichnet, dass** die Faltlinien (9, 10) parallel zueinander verlaufen.

3. Mehrfachfaltpackung nach einem der vorangehenden Ansprüche, **dadurch gekennzeichnet, dass** jeder Teilbereich (6, 7) gleich viele Fächer (2, 2a) aufweist.

4. Mehrfachfaltpackung nach einem der vorangehenden Ansprüche, **dadurch gekennzeichnet, dass** jeder Teilbereich (6, 7) gegenüber dem Standbereich (8) jeweils um etwa 90° schwenkbar ist.

5. Mehrfachfaltpackung nach einem der vorangehenden Ansprüche, **dadurch gekennzeichnet, dass** sich der Standbereich (8) mittig auf der Mehrfachfaltpackung (1) befindet.

6. Mehrfachfaltpackung nach einem der vorangehenden Ansprüche, **dadurch gekennzeichnet, dass** in einem Aufstellzustand der Mehrfachfaltpackung (1) der erste Teilbereich (6) parallel zum zweiten Teilbereich (7) angeordnet ist.

7. Mehrfachfaltpackung nach einem der vorangehenden Ansprüche, **dadurch gekennzeichnet, dass** mindestens eine der beiden Faltlinien (9, 10) eine Perforation aufweist.

8. Mehrfachfaltpackung nach einem der vorangehenden Ansprüche, **dadurch gekennzeichnet, dass** zwischen allen benachbarten Fächern (2, 2a) jeweils eine Perforation (11) vorgesehen ist.

9. Mehrfachfaltpackung nach einem der Ansprüche 8 oder 9, **dadurch gekennzeichnet, dass** zwischen zwei benachbarten Löchern der Perforation (11) Stege mit einer Breite von 0,5 bis 1,0 mm vorgesehen sind.

10. Mehrfachfaltpackung nach einem der vorangehenden Ansprüche, **dadurch gekennzeichnet, dass** ein Verschluss (12) zum lösbaren Halten der Mehrfachfaltpackung (1) in einem geschlossenen Zustand vorgesehen ist.

11. Mehrfachfaltpackung nach Anspruch 10, **dadurch gekennzeichnet, dass** als Verschlusselement der Mehrfachfaltpackung (1) mindestens ein Etikett (19) vorgesehen ist.

12. Verpackungsmaschine zum Herstellen einer Mehrfachfaltpackung (1) nach einem der vorangehenden Ansprüche, umfassend eine Einrichtung zum Erzeugen zweier Faltlinien (9, 10) in der Mehrfachfaltpackung (1), insbesondere zweier paralleler Faltlinien (9, 10).

13. Verpackungsmaschine nach Anspruch 12, **dadurch gekennzeichnet, dass** zwei parallel angeordnete Stanzmesser oder Rotationsmesser zum Erzeugen einer Perforation in den Faltlinien (9, 10) vorgesehen sind.

14. Verpackungsmaschine nach einem der Ansprüche 12 oder 13, **dadurch gekennzeichnet, dass** ein Umfaltwerkzeug zum automatischen Umfalten der ersten (6) und/oder zweiten Teilbereiche (7) der Mehrfachfaltpackung (1) um die Faltlinien (9, 10) vorgesehen ist.
